(19) Europäisches Patentamt
European Patent Office
Office européen des brevets

(11) Publication number: **0 503 079 A1**

(12) **EUROPEAN PATENT APPLICATION published in accordance with Art. 158(3) EPC**

(21) Application number: **91916784.1**

(51) Int. Cl.5: **C07D 401/12**, A61K 31/50

(22) Date of filing: **30.09.91**

(86) International application number: **PCT/JP91/01316**

(87) International publication number: **WO 92/06087 (16.04.92 92/09)**

(30) Priority: **02.10.90 JP 266034/90**

(43) Date of publication of application: **16.09.92 Bulletin 92/38**

(84) Designated Contracting States: **AT BE CH DE DK ES FR GB GR IT LI LU NL SE**

(71) Applicant: **KAKEN PHARMACEUTICAL CO., LTD.**
**No. 28-8, 2-chome, Honkomagome**
**Bunkyo-Ku**
**Tokyo 113(JP)**

(72) Inventor: **NAKANISHI, Michio**
**393-1, Kitahorikawa**
**Nakatsu-shi, Oita 871(JP)**
Inventor: **UCHIDA, Katsuhiro**
**336, Gojoagaru Kashiwaya-cho, Yanaginobanbadori**
**Shimogyo-ku, Kyoto-shi, Kyoto 600(JP)**
Inventor: **NAKANO, Jun**
**1180-5, Katsube-cho**
**Moriyama-shi Shiga 524(JP)**
Inventor: **NAGAHARA, Michiko**
**591, Oaza Nagahara, Yasu-cho**
**Yasu-gun, Shiga 520-23(JP)**
Inventor: **TANAKA, Kiyomi**
**621-25, Oishihigashi-cho**
**Otsu-shi, Shiga 520-22(JP)**
Inventor: **YAJIMA, Motoyuki**
**Kosumo Moriyama 3bankan 601 896-1, Moriyama-cho**
**Moriyama-shi Shiga 524(JP)**
Inventor: **NAKAGAWA, Terutake**
**Haitsu Yamanokami 105, 1-4, Ichiriyama 3-chome**
**Otsu-shi, Shiga 520-21(JP)**
Inventor: **MURATA, Takahiko, Kimura Daiichi Bldg.**
**401, 50-5, Takehana Takenokaido-cho**
**Yamashina-ku, Kyoto-shi, Kyoto 607(JP)**
Inventor: **OKAMOTO, Taira**
**10-40, Kojogaoka**
**Otsu-shi Shiga 520(JP)**

(74) Representative: **Türk, Dietmar, Dr. rer. nat. et al**
**Türk, Gille, Hrabal, Leifert Patentanwälte**
**Brucknerstrasse 20**
**W-4000 Düsseldorf 13(DE)**

(54) **PYRIDAZINONE-SUBSTITUTED ETHYNYLPHENYL DERIVATIVE AND REMEDY FOR CIRCULATORY ORGAN DISEASE CONTAINING THE SAME AS ACTIVE INGREDIENT.**

(57) Racemic or enantiomeric pyridazinone-substituted ethynylphenyl derivative represented by general formula (I), pharmacologically acceptable salt thereof, and remedy for circulatory organ diseases, thrombosis and renal disease. In the said formula, $R^1$ represents hydrogen, $C_1$ to $C_6$ alkyl, $C_1$ to $C_4$ alkoxycarbonyl, substituted amino $C_1$ to $C_6$ alkyl, hydroxy $C_1$ to $C_6$ alkyl, or phenyl substituted with alkyl, alkoxy, nitro or halogen; $R^2$ represents hydrogen, $C_1$ to $C_4$ alkoxy or halogen; $R^3$ represents $C_1$ to $C_4$ alkoxy, $C_1$ to $C_6$ alkylamino or $C_1$ to $C_4$ alkoxy $C_1$ to $C_4$ alkoxy; $R^4$ represents $C_1$ to $C_4$ alkyl or amino; $R^5$ represents hydrogen or $C_1$ to $C_4$ alkyl; $R^6$ represents

EP 0 503 079 A1

hydrogen or $C_1$ to $C_4$ alkyl; and $R^7$ represents hydrogen or $C_1$ to $C_4$ alkyl.

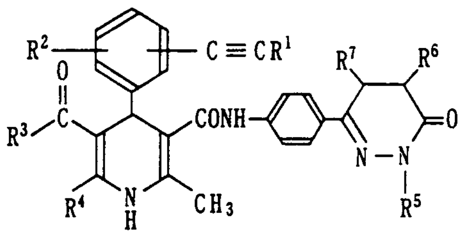

(I)

## TECHNICAL FIELD

The present invention is a useful invention in the medicinal field. Further detailedly, the present invention relates to ethynylphenyl derivative substituted by pyridazinone, process for preparing the same and medicament for circulatory disease having therapeutic effect for arteriosclerosis and/or hypertension, stenocardia, cardiac failure and/or peripheral circulatory disease, or medicament for kidney disease containing the same as an effective ingredient.

## BACKGROUND ART

Hitherto, 1,4-dihydropyridines have been known as compounds having pharmacological activities such as antihypertensive activity and vasodilative activity. For example, dimethyl 2,6-dimethyl-4-(2-nitrophenyl)-1,4-dihydropyridine-3,5-dicarboxylate (hereinafter referred to as "nifedipine") is described in USP 3644627 and 2-(N-benzyl-N-methylamino) ethyl, methyl 2,6-dimethyl-4-(3-nitrophenyl)-1,4-dihydropyridine-3,5-dicarboxylate hydrochloride (hereinafter referred to as "nicardipine") is described in USP 3985758. Pyridazinones have been known as compounds having pharmacological activities such as anticoagulative activity and activity of improving cardiac contractile force. For example, imazodan is described in USP 4353905. A compound wherein side chain of pyridazinone is introduced into 1,4-dihydropyridine skeleton is described in Japanese Unexamined Patent Publication No. 145180/1986 and its pharmacological characteristic is to have vasodilative activity, anticoagulative activity and activity of improving cardiac contractile force.

However, compounds wherein substituents of phenyl group at the 4-position of 1,4-dihydropyridine include ethynyl group are not described in Japanese Unexamined Patent Publication No. 145180/1986. Although many 1,4-dihydropyridine compounds have been reported, 4-ethynylphenyl derivative has not been reported. Further, although many pyridazinones have been reported, the compound linked to dihydropyridine is described only in the above-mentioned Japanese Unexamined Patent Publication No. 145180/1986. Therefore, the compound of the present invention is quite a new compound in structure.

The present invention is directed to provide a compound having therapeutic effect for any of hypertension, cerebrocirculatory disease, thrombosis and concomitant diseases thereof, for example, kidney diseases such as nephritis.

The compound of the present invention is a novel compound. The pharmacological characteristic due to its novelty is to have not only the pharmacological characteristics of the above-mentioned known compounds but also new pharmacological characteristics. That is, the activity to sympathetic nervous system is not described in USP 4353905. Though the compound of the present invention does not have a potent calcium antagonistic activity, the compound shows antinorepinephrine activity as shown in Test Example 3. The compound of the present invention has remarkable cerebrocirculation improving activity as shown in Test Example 2, though there is no description of concrete example in Japanese Unexamined Patent Publication No. 145180/1986. Further, the compound has platelet aggregation inhibiting activity as shown in Test Example 4. Therefore, the compound posesses preferable pharmacological characteristics as an circulation improving agent. The compound of the present invention has, as shown in Test Example 5, a potent stress ulcers inhibiting activity which activity is not at all described in Japanese Unexamined Patent Publication No. 145180/1986. Patients generally have a great mental burden due to diseases, which burden often causes supervention of stress ulcers. It is obvious in the practical therapy that the supervened disease forces greater physical and mental burden on patients, so a vicious circle of diseases is accelerated. The compound of the present invention has also a kidney disease inhibiting activity as shown in Test Example 7.

Therefore, the compound of the present invention is useful for clinical therapy of hypertension, cerebrocirculatory disease, thrombosis and concomitant diseases thereof, for example, kidney diseases such as nephritis, because of having potent smooth muscle relaxing and platelet aggregation inhibiting activity. Further, the compound of the present invention is extremely useful as a medicament having a stress ulcers inhibiting activity.

## DISCLOSURE OF THE INVENTION

As a result of the continuous effort and detailed investigation of the present inventors, an ethynylphenyl derivative substituted by pyridazinone having the formula (I):

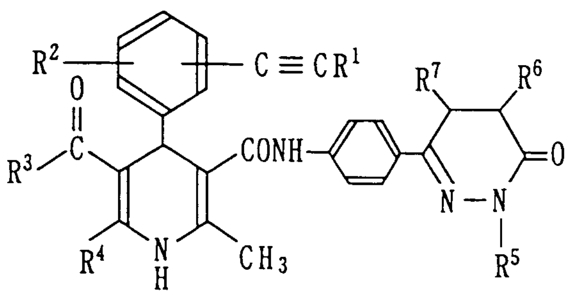

(I)

wherein $R^1$ is hydrogen atom, a $C_{1-6}$ alkyl group, a $C_{1-4}$ alkoxycarbonyl group, a substituted amino $C_{1-6}$ alkyl group, a hydroxy $C_{1-4}$ alkyl group or a phenyl group substituted by an alkyl group, an alkoxy group, nitro group or a halogen atom, $R^2$ is hydrogen atom, a $C_{1-4}$ alkoxyl group, or a halogen atom, $R^3$ is a $C_{1-4}$ alkoxyl group, a $C_{1-6}$ alkylamino group or a $C_{1-4}$ alkoxy $C_{1-4}$ alkoxyl group, $R^4$ is a $C_{1-4}$ alkyl group or amino group, $R^5$ is hydrogen atom or a $C_{1-4}$ alkyl group, $R^6$ is hydrogen atom or a $C_{1-4}$ alkyl group, and $R^7$ is hydrogen atom or a $C_{1-4}$ alkyl group, which may be a racemate or an optical isomer, or a pharmacologically acceptable salt thereof has been successfully synthesized. It has been also found that the above-mentioned derivative has excellent vasodepressor activity, a cerebral blood flow increasing activity, a coronary blood flow increasing activity, a platelet aggregation inhibiting activity, stress ulcers inhibiting activity, an antinorepinephrine activity and a phosphodiesterase (PDE) inhibiting activity. That is, it has been found that the above-mentioned derivative is useful as a medicament for prevention and therapy of hypertension, cerebrocirculatory disease, cardiac failure or various diseases caused by platelet aggregation, or a medicament for therapy of kidney disease and is excellent in safety. Consequently, the present invention has been accomplished.

That is, the present invention relates to ① an ethynylphenyl derivative substituted by pyridazinone having the formula (I):

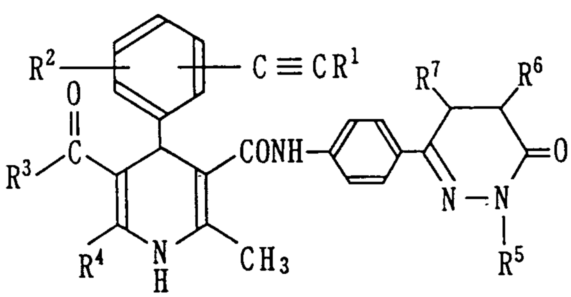

(I)

wherein $R^1$ is hydrogen atom, a $C_{1-6}$ alkyl group, a $C_{1-4}$ alkoxycarbonyl group, a substituted amino $C_{1-6}$ alkyl group, a hydroxy $C_{1-4}$ alkyl group or a phenyl group substituted by an alkyl group, an alkoxyl group, nitro group or a halogen atom, $R^2$ is hydrogen atom, a $C_{1-4}$ alkoxyl group, or a halogen atom, $R^3$ is a $C_{1-4}$ alkoxyl group, a $C_{1-6}$ alkylamino group or a $C_{1-4}$ alkoxy $C_{1-4}$ alkoxyl group, $R^4$ is a $C_{1-4}$ alkyl group or amino group, $R^5$ is hydrogen atom or a $C_{1-4}$ alkyl group, $R^6$ is hydrogen atom or a $C_{1-4}$ alkyl group, and $R^7$ is hydrogen atom or a $C_{1-4}$ alkyl group, which may be a racemate or an optical isomer, or a pharmacologically acceptable salt thereof, ② a medicament for a circulatory disease comprising the above-mentioned ethynylphenyl derivative substituted by pyridazinone or the pharmacologically acceptable salt thereof as an effective ingredient, ③ a medicament for thrombosis comprising the above-mentioned ethynylphenyl derivative substituted by pyridazinone or the pharmacologically acceptable salt thereof as an effective ingredient and ④ a medicament for a kidney disease comprising the above-mentioned ethynylphenyl derivative substituted by pyridazinone or the pharmacologically acceptable salt thereof as an effective ingredient.

In detail, a $C_{1-6}$ alkyl group represented by $R^1$ in the above-mentioned formula (I) is any of a straight chain, a branched chain and a ring and there are, for example, methyl, ethyl, propyl, isopropyl, butyl, isobutyl, sec-butyl, t-butyl, pentyl, isopentyl, neopentyl, hexyl, cyclopropyl, cyclobutyl, cyclopentyl, cyclohexyl and the like. A $C_{1-4}$ alkoxycarbonyl group comprises a $C_{1-4}$ alkoxyl group which may be any of a straight chain, a branched chain and a ring and there are, for example, methoxycarbonyl, ethoxycarbonyl, propoxycarbonyl, isopropoxycarbonyl, butoxycarbonyl, isobutoxycarbonyl, sec-butoxycarbonyl, t-butoxycarbonyl, cyclopropoxycarbonyl and the like. A substituted amino $C_{1-6}$ alkyl group comprises amino group substituted by one or two $C_{1-3}$ alkyl groups or a substituted amino group having the formula:

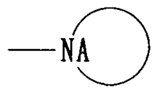

wherein N is nitrogen atom of amino group, ring A contains 4 to 5 carbon atom and may contain, if necessary, one oxygen atom to form a heterocycle, and a $C_{1-6}$ alkyl group. As the above-mentioned substituted amino $C_{1-6}$ alkyl group, there are, for example, methylaminomethyl, methylaminoethyl, methylaminobutyl, methylaminopentyl, ethylaminomethyl, ethylaminoethyl, propylaminomethyl, dimethylaminoethyl, diethylaminomethyl, ethylaminoethyl, ethylaminopropyl, ethylaminobutyl, ethylaminopentyl, propylaminomethyl, propylaminoethyl, propylaminopropyl, dimethylaminomethyl, dimethylaminoethyl, dimethylaminopropyl, dimethylaminobutyl, dimethylaminopentyl, diethylaminomethyl, diethylaminoethyl, diethylaminopropyl, diethylaminobutyl, diethylaminopentyl, morpholinomethyl, 2-morpholinoethyl, 3-morpholinopropyl, 4-morpholinobutyl, 5-morpholinopentyl, piperidinomethyl, 2-piperidinoethyl, 3-piperidinopropyl, 4-piperidinobutyl, 5-piperidinopentyl, piperazinylmethyl, 2-piperazinylethyl, 3-piperazinylpropyl, 4-piperazinylbutyl, 5-piperazinylpentyl, N,N-ethylmethylaminopropyl and the like. A hydroxy $C_{1-4}$ alkyl group comprises a $C_{1-4}$ alkyl group and there are, for example, hydroxymethyl, hydroxyethyl, hydroxypropyl, hydroxybutyl and the like. A substituted phenyl group is a phenyl group which is substituted by a $C_{1-4}$ alkyl group, a $C_{1-4}$ alkoxyl group, nitro group or a halogen atom at any position of the ring. There are, for example, p-nitrophenyl group, o-methylphenyl group, p-chlorophenyl group and the like. A $C_{1-4}$ alkoxyl group represented by $R^2$ is any of a straight chain, a branched chain and a ring and there are, for example, methoxy, ethoxy, propoxy, isopropoxy, butoxy, isobutoxy, sec-butoxy, t-butoxy, cyclopropoxy and the like. As a halogen atom, there are, for example, fluorine, chlorine, bromine, iodine and the like. A $C_{1-4}$ alkoxyl group represented by $R^3$ is any of a straight chain, a branched chain and a ring and there are, for example, methoxy, ethoxy, propoxy, isopropoxy, butoxy, isobutoxy, sec-butoxy, t-butoxy, cyclopropoxy and the like. As a $C_{1-6}$ alkylamino group, there are, for example, methylamino, ethylamino, diethylamino, ethylmethylamino, propylamino, methylpropylamino, ethylpropylamino, cyclopropylamino, dicyclopropylamino, butylamino, pentylamino, methylpentylamino and the like. As a $C_{1-4}$ alkoxy $C_{1-4}$ alkoxyl group, there are, for example, methoxymethoxy, methoxyethoxy, methoxypropoxy, methoxybutoxy, ethoxymethoxy, ethoxyethoxy, ethoxypropoxy, ethoxybutoxy, propoxymethoxy, propoxyethoxy, propoxypropoxy, propoxybutoxy and the like. As a $C_{1-4}$ alkyl group represented by $R^4$, there are, for example, methyl, ethyl, propyl, isopropyl and the like, and methyl is preferable. As an amino group, a primary amino group is exemplified. As a $C_{1-4}$ alkyl group represented by $R^5$, $R^6$ or $R^7$, there are, for example, methyl, ethyl, propyl, isopropyl and the like.

The compound of the present invention can be changed into the acid addition salts thereof by known means and can be present as a salt. The salt is not particularly limited if it is pharmacologically acceptable. For example, there are an acid addition salt of a mineral acid such as hydrochloric acid, hydrobromic acid, hydroiodic acid or sulfuric acid, an acid addition salt of an organic acid such as methanesulfonic acid, p-toluenesulfonic acid, benzenesulfonic acid, acetic acid, phosphoric acid, oxalic acid, maleic acid, tartaric acid, citric acid, gluconic acid or lactic acid, and the like.

There exists asymmetric carbon atom in the compound of the present invention. The present invention includes optical isomers due to asymmetric carbon or a mixture thereof mixed in an optional rate besides racemic compounds.

Process for preparation A

In a typical process for preparation, the ethynylphenyl derivative having the formula (I) described in the reaction formula (a) can be prepared by adding an ethynylbenzaldehyde derivative (II), an acetoamide derivative (III) and a $\beta$-aminoacrylic acid derivative (IV) to a suitable organic solvent, for example, a lower alkanol such as ethanol, namely, by Hantzsch synthesis method.

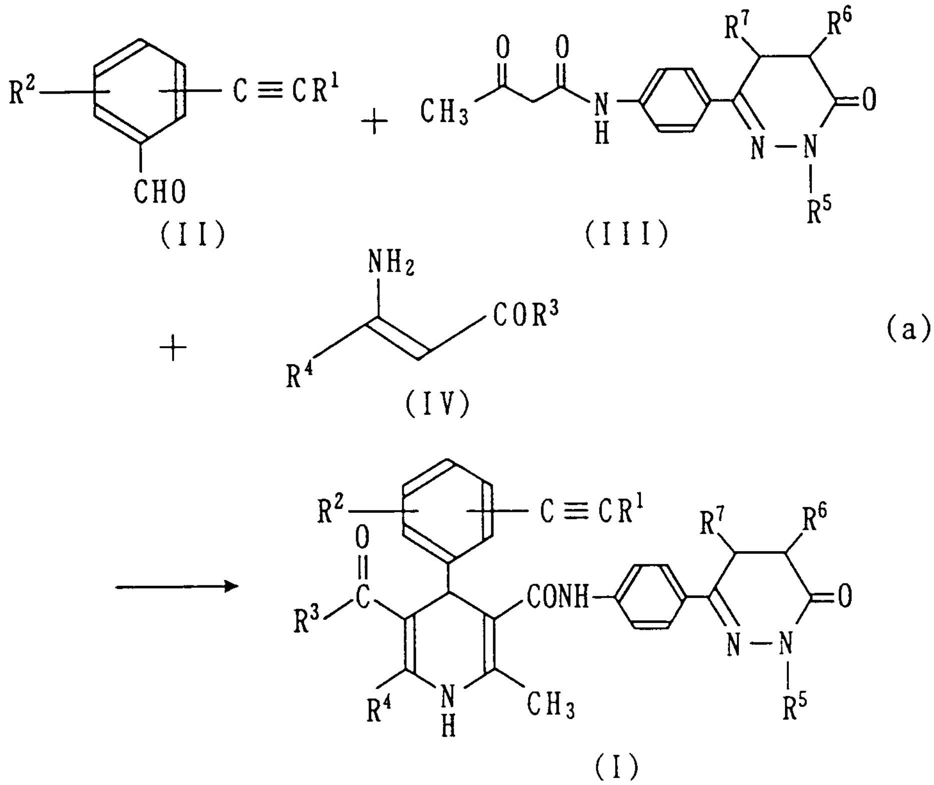

(II) (III) (IV) (a) (I)

In the formulae, $R^1$ is hydrogen atom, a $C_{1-6}$ alkyl group, a $C_{1-4}$ alkoxycarbonyl group, a substituted amino $C_{1-6}$ alkyl group, a hydroxy $C_{1-4}$ alkyl group or a phenyl group substituted by an alkyl group, an alkoxyl group, nitro group or a halogen atom, $R^2$ is hydrogen atom, a $C_{1-4}$ alkoxyl group or a halogen atom, $R^3$ is a $C_{1-4}$ alkoxyl group, a $C_{1-6}$ alkylamino group or a $C_{1-4}$ alkoxy $C_{1-4}$ alkoxyl group, $R^4$ is a $C_{1-4}$ alkyl group or amino group, $R^5$ is hydrogen atom or a $C_{1-4}$ alkyl group, $R^6$ is hydrogen atom or a $C_{1-4}$ alkyl group and $R^7$ is hydrogen atom or a $C_{1-4}$ alkyl group.

Organic solvents which can be used in the present invention are not particularly limited, if the solvents do not remarkably inhibit this type of reaction. As the solvents, lower alkanols such as ethanol, methanol, isopropyl alcohol and n-propyl alcohol are preferable.

With respect to the used amount of each reactant in the present reaction, 1 to 1.5 equivalents of an acetamide derivative (III) and 1 to 1.5 equivalents of a $\beta$-aminoacrylic acid derivative (IV) are preferably used per equivalent of an ethynylbenzaldehyde derivative (II). In particular, 1 to 1.2 equivalents are preferably used.

The reaction temperature is preferably 20° to 120°C, in particular, preferably 30° to 100°C and the reaction time is preferably 1 to 24 hours, in particular, preferably 1 to 20 hours.

After the above-mentioned reaction, the compound having the formula (I) can be purified and isolated by means of a conventional treatment method such as recrystallization or chromatography.

Process for preparation B

In a typical process for preparation, the ethynylphenyl derivative having the formula (I) described in the reaction formula (b) can be prepared by adding an ethynylbenzaldehyde derivative (II), a $\beta$-dicarbonyl derivative (V) and a $\beta$-aminocrotoamide derivative (VI) to a suitable organic solvent such as a lower alkanol, e.g. ethanol.

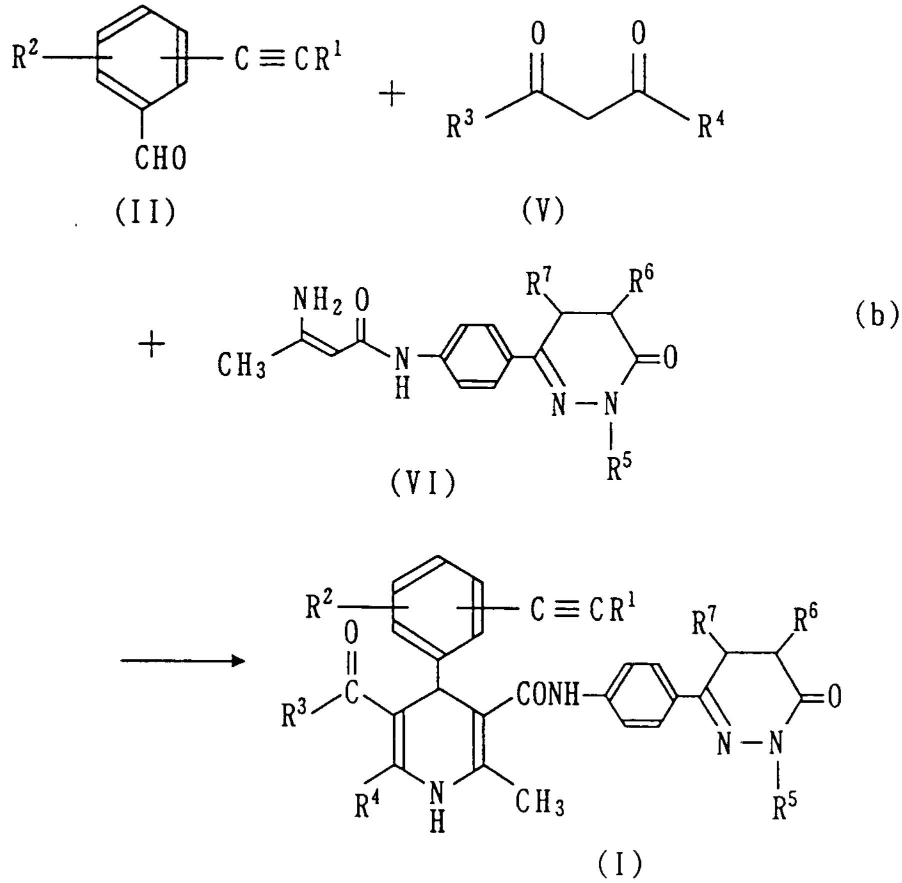

(II) (V)

(VI)

(b)

(I)

In the formulae, $R^1$, $R^2$, $R^3$, $R^4$, $R^5$, $R^6$ and $R^7$ are as defined above.

Organic solvents which can be used in the present reaction are not particularly limited, if the solvents do not remarkably inhibit this type of reaction. As these solvents, lower alkanols such as ethanol, methanol, isopropyl alcohol, n-propyl alcohol are preferable.

With respect to the used amount of each reactant in the present reaction, 1 to 1.5 equivalents of a $\beta$-dicarbonyl derivative (V) and 1 to 1.5 equivalents of a $\beta$-aminocrotoamide derivative (VI) are preferably used per equivalent of an ethynylbenzaldehyde derivative (II). In particular, 1 to 1.2 equivalents are preferably used.

The reaction temperature is preferably 20° to 120°C, in particular, preferably 30° to 100°C and the reaction time is preferably 1 to 24 hours, in particular, preferably 1 to 20 hours.

After the above-mentioned reaction, the compound having the formula (I) can be purified and isolated by means of a conventional treatment method such as recrystallization or chromatography.

Process for preparation C

In a typical process for preparation, the ethynylphenyl derivative having the formula (I) described in the reaction formula (c) can be prepared by adding a benzylidene derivative (VII) and a $\beta$-aminoacrylic acid derivative (IV) or a hydrochloride thereof with adding, if the hydrochloride is used, an alkoxide to a suitable organic solvent such as a lower alkanol, e.g. ethanol.

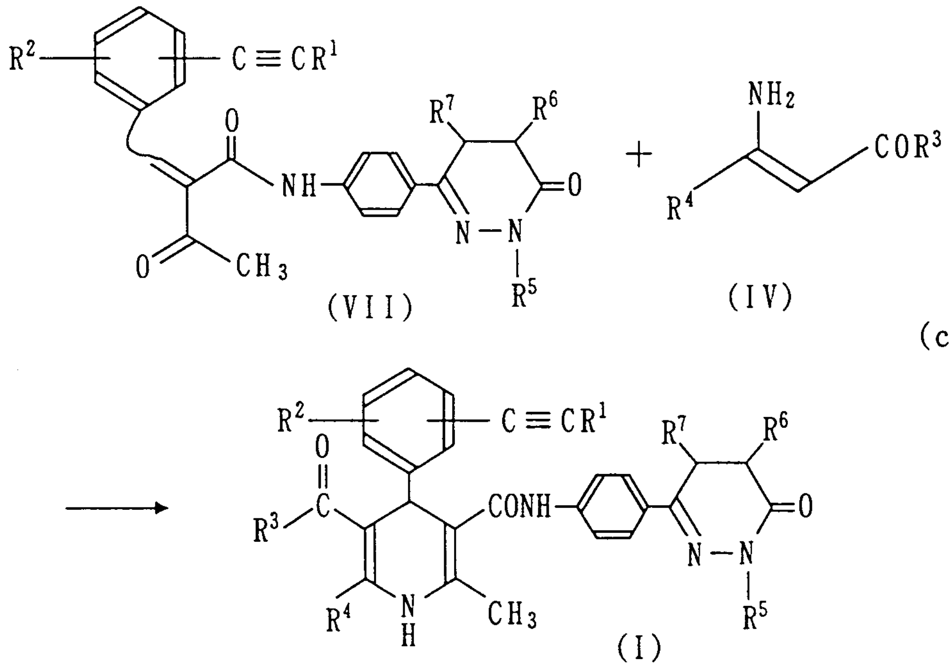

(VII) (IV) (c)

(I)

In the formulae, $R^1$, $R^2$, $R^3$, $R^4$, $R^5$, $R^6$ and $R^7$ are as defined above.

Organic solvents which can be used in the present reaction are not particularly limited, if the solvents do not remarkably inhibit this type of reaction. As these solvents, lower alkanols such as ethanol, methanol, isopropyl alcohol, n-propyl alcohol are preferable.

With respect to the used amount of each reactant in the present reaction, 1 to 1.5 equivalents of a β-aminoacrylic acid derivative (IV) is preferably used per equivalent of a benzylidene derivative (VII). In particular, 1 to 1.2 equivalents are preferably used. When the hydrochloride is used, an equivalent of the hydrochloride is preferably used.

The reaction temperature is preferably 20° to 120°C, in particular, preferably 30° to 100°C and the reaction time is preferably 0.5 to 24 hours, in particular, preferably 0.5 to 20 hours.

After the above-mentioned reaction, the compound having the formula (I) can be purified and isolated by means of a conventional treatment method such as recrystallization or chromatography.

Process for preparation D

In a typical process for preparation, the ethynylphenyl derivative having the formula (I) described in the reaction formula (d) can be prepared by reacting a carboxylic acid derivative (VIII) or its reactive derivative with an aniline derivative (IX) in a suitable inactive organic solvent according to a known method.

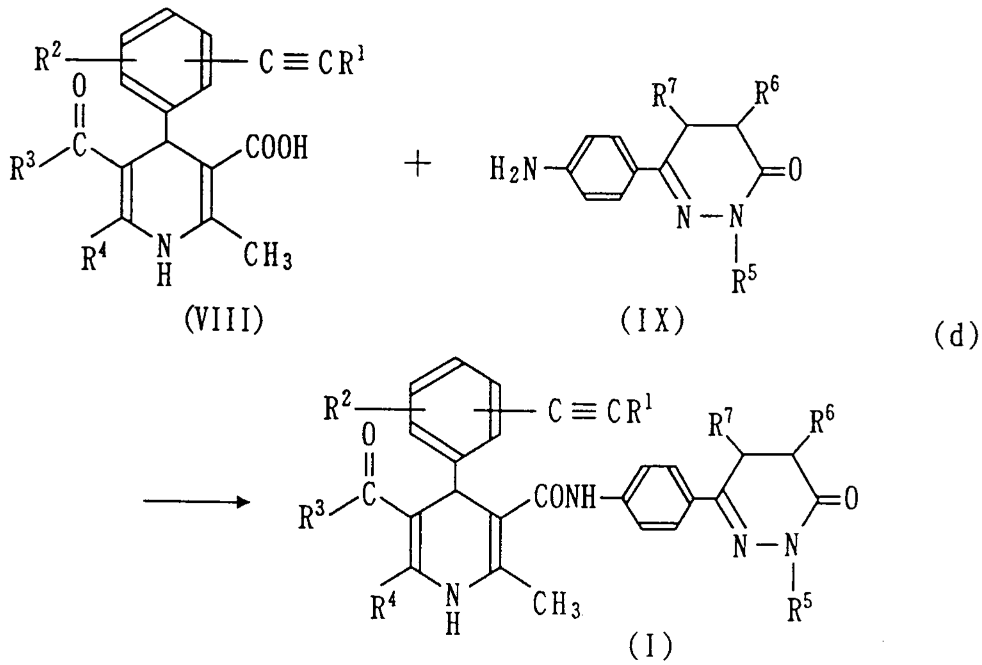

(VIII) (IX) (d)

(I)

In the formulae, $R^1$, $R^2$, $R^3$, $R^4$, $R^5$, $R^6$ and $R^7$ are as defined above.

Organic solvents which can be used in the present reaction are not particularly limited, if the solvents do not remarkably inhibit this type of reaction. As these solvents, for example, chlorinated hydrocarbons such as methylene chloride, chloroform, tetrachloromethane and 1,2-dichloroethane, ethers such as diethylether, tetrahydrofuran, dioxane and 1,2-dimethoxyethane, toluene, xylene, dimethylformamide, hexamethylphosphorotriamide and the like are preferable. Condensing agents are not particularly limited, if the agents have dehydrating activity. As these condensing agents, dicyclohexylcarbodiimide and carbonyldiimidazole are particularly preferable.

With respect to the used amount of each reactant in the present reaction, 1 to 1.5 equivalents of a phenylamine derivative (IX) is preferably used per equivalent of a carboxylic acid derivative (VIII). In particular, 1 to 1.2 equivalents are preferably used. The reaction temperature is preferably -70° to 100°C, in particular, preferably 20° to 100°C and the reaction time is preferably 1 to 24 hours, in particular, preferably 1 to 20 hours.

After the above-mentioned reaction, the compound having the formula (I) can be purified and isolated by means of a conventional treatment method such as recrystallization or chromatography.

BEST MODE FOR CARRYING OUT THE INVENTION

As the compound having the above-mentioned formula (I) obtainable according to the present invention, the compounds illustrated in Table 1 can be exemplified.

Table 1

| Compound | $R^1$ | $R^2$ | $R^3$ | $R^4$ | $R^5$ | $R^6$ | $R^7$ |
|---|---|---|---|---|---|---|---|
| 1 | H | H | -OMe | Me | H | H | H |
| 2 | Me | H | -OMe | Me | H | H | H |
| 3 | $-(CH_2)_4CH_3$ | H | -OMe | Me | H | H | H |
| 4 | $-PhNO_2$ *1 | H | -OMe | Me | H | H | H |
| 5 | $-(CH_2)_4Mor$ *2 | H | -OMe | Me | H | H | H |
| 6 | $-(CH_2)_2OH$ | H | -OMe | Me | H | H | H |

- continued -

- continued -

| Compound | $R^1$ | $R^2$ | $R^3$ | $R^4$ | $R^5$ | $R^6$ | $R^7$ |
|---|---|---|---|---|---|---|---|
| 7 | $-C(CH_3)_2OH$ | H | -OMe | Me | H | H | H |
| 8 | $-CO_2C(CH_3)_3$ | H | -OMe | Me | H | H | H |
| 9 | H | Cℓ | -OMe | Me | H | H | H |
| 10 | H | H | -NH cPr *3 | Me | H | H | H |
| 11 | H | H | -N(Pr *4)(Me) | Me | H | H | H |
| 12 | H | H | -OEt | $NH_2$ | H | H | H |
| 13 | H | H | -OMe | Me | Me | H | H |
| 14 | H | H | -OMe | Me | Et | H | H |
| 15 | H | H | -OMe | Me | H | Me | H |
| 16 | H | H | -OMe | Me | H | H | Me |
| 17 | $-C(CH_3)_2OH$ | OMe | -OEt | Me | H | H | H |
| 18 | H | OMe | -OEt | Me | H | H | H |
| 19 | H | H | -OMe | Me | H | H | Et |
| 20 | H | H | -OEt | Me | H | H | H |
| 21 | H | H | -OMe | Me | H | H | Pr *4 |
| 22 | H | H | $-OCH_2CH_2OMe$ | Me | H | H | H |
| 23 | H | H | -OEt | $NH_2$ | H | H | Me |
| 24 | $-C(CH_3)_2OH$ | H | -OEt | $NH_2$ | H | H | H |
| 25 | H | H | $-OCH_2CH_2OMe$ | $NH_2$ | H | H | Et |
| 26 | H | H | -NH cPr *3 | $NH_2$ | H | Me | H |

[Note 1] In all cases except for the compound 9, ethynyl group is substituted at the 3-position of the 4-phenyl ring in the 1,4-dihydropyridine skeleton relative to the dihydropyridine ring. Only the compound 9 has ethynyl group at the 2-position and chlorine at the 3-position.

[Note 2] *1: p-Nitrophenyl group
*2: Morpholino group
*3: Cyclopropyl group
*4: n-Propyl group

Test Example 1

Activity on blood pressure of spontaneously hypertensive rats (SHR)

In each group, 3 male SHR rats (13 weeks) were employed. Each test compound was suspended or dissolved in a 0.5 % methyl cellulose solution. After the concentration was adjusted, the dose became 30 mg/kg, the solution was administered orally. Each rat was previously kept warm at 40 °C for 10 minutes, and then, the caudal vein pressure of rats was measured by a tail cuff method using sphygmanometer (made by Muromachi Kikai Co., Ltd.) before the administration, and one hour and three hours after the administration.

Change of the blood pressure was calculated by the following formula.

Change rate (%) = [(Measured value after administration - Measured value before administration) / Measured value before administration] x 100

The results are shown in Table 2.

Table 2

| Compound No. | Change rate of blood pressure (%) | |
|---|---|---|
| | 1 hr. value | 3 hr. value |
| 1 | −35.5 | −25.8 |
| 3 | −5.2 | −3.2 |
| 4 | −1.2 | −0.2 |
| 5 | −0.9 | −2.7 |
| 6 | 2.6 | 2.1 |
| 7 | −4.3 | −8.1 |
| 8 | −2.2 | −2.9 |
| 10 | −0.6 | −2.5 |
| 12 | −10.5 | −12.2 |

− continued −

- continued -

| Compound No. | Change rate of blood pressure (%) | |
|---|---|---|
| | 1 hr. value | 3 hr. value |
| 13 | 3.3 | 3.0 |
| 15 | −22.6 | −25.2 |
| 16 | −36.3 | −36.7 |
| 17 | −5.3 | 7.9 |
| 18 | −17.1 | −10.1 |
| 19 | −23.7 | −28.2 |
| 20 | −44.5 | −50.9 |
| 21 | −4.5 | −6.7 |
| 22 | −32.3 | −26.6 |
| 23 | −46.7 | −38.7 |
| 24 | −15.4 | −10.3 |
| 25 | −5.9 | −3.1 |
| 26 | −3.0 | −1.4 |
| 0.5 % methylcellulose solution (Control) | 1.8 | −6.8 |

Test Example 2

Activity on hypoxia induced in decapitation of mice

In each group, 3 Slc-ddy male mice (4 weeks) were employed. Each test compound was suspended or dissolved in a 0.5 % methyl cellulose solution. After the concentration was adjusted, the dose became 100 mg/g, the solution was administered orally. One hour after the administration, mice were decapitated and gasping time was measured.

Inhibition rate was calculated by the following formula.

$$\text{Inhibition rate} = \frac{\text{Mean gasping time of administration group} - \text{Mean gasping time of control group}}{\text{Mean gasping time of control group}} \times 100$$

The results are shown in Table 3.

Table 3

| Compound No. | | Inhibition rate (%) |
|---|---|---|
| 1 | | 28.6 |
| 4 | | 13.2 |
| 5 | | 3.2 |
| 6 | | 21.5 |
| 7 | | 19.1 |
| 8 | | 12.8 |
| 12 | | 30.2 |
| 15 | | 5.2 |
| 16 | | 3.4 |
| 17 | | 25.8 |
| 19 | | 5.6 |
| 24 | | 24.2 |
| 25 | | 28.3 |
| 0.5 % methylcellulose solution (Control) | 1.8 | 0.0 |

Test Example 3

Anti-norepinephrine activity

Aortae abdominalis of male Wister rats (8-12 weeks) were delivered and cut in spiral-shape to be strip preparations. Each test compounds was disolved in DMSO. Then, the solution was diluted with distilled water and prepared to be desired concentration. Using an auto-Magnus apparatus, as to the vasoconstriction after adding norepinephrine, contractive forces were measured under the existence of the test compounds. The results are shown in Table 4.

Table 4

| | | Contractive force*1 | | |
|---|---|---|---|---|
| Concentration of compound (M) | | $10^{-9}$ | $10^{-8}$ | $10^{-7}$ |
| Compound No. | 1 | 102.8 | 87.4 | 15.7 |
| | 3 | 92.4 | 68.3 | 12.5 |
| | 4 | 93.2 | 71.5 | 13.2 |
| | 6 | 90.5 | 70.2 | 10.8 |
| | 7 | 100.3 | 89.2 | 16.3 |
| | 12 | 90.2 | 69.4 | 10.3 |
| | 15 | 85.6 | 68.3 | 9.8 |
| | 16 | 105.3 | 86.4 | 17.9 |
| | 17 | 87.7 | 70.1 | 8.9 |
| | 18 | 111.2 | 90.5 | 16.3 |
| | 19 | 101.1 | 84.9 | 14.9 |
| | 24 | 88.8 | 72.4 | 11.1 |
| | 25 | 91.5 | 70.9 | 12.1 |

*1 The values show the tension under the existence of the test compound when the tension by norepinephrine ($10^{-7}$ M) is regarded as 100.

Test Example 4

Inhibitory activity on platelet aggregation

Blood was exsanguinated from New Zealand White male rabbits weighing from 2.5 to 3.0 kg and subjected to the test. Each test compound was dissolved in ethanol and prepared to be 1 x $10^{-5}$, 1 x $10^{-6}$ or 1 x $10^{-7}$ M respectively. Each aggregation response was measured by turbidimetry. Concentration of aggregating agent was 2 µg/mℓ of collagen, 0.6 µg/mℓ of adenosine 5'-diphosphate (ADP) or 5 x $10^{-9}$ M of platelet aggregating factor (PAF).

Inhibition rate was calculated by the following formula.

$$\text{Inhibition rate} = \frac{\text{Mean aggregation rate of control group} - \text{Mean aggregation rate of administration group}}{\text{Mean aggregation rate of control group}} \times 100$$

The results are shown in Table 5.

Table 5

| Inhibition rate (%) | | | | | | | |
|---|---|---|---|---|---|---|---|
| | | ADP aggregation | | | Collagen aggregation | | PAF aggregation |
| Concentration of compound (M) | | $10^{-7}$ | $10^{-6}$ | $10^{-5}$ | $10^{-7}$ | $10^{-6}$ | $10^{-5}$ |
| Compound No. | 1 | 21 | 70 | 94 | 21 | 94 | 64 |
| | 3 | 18 | 59 | 87 | 28 | 93 | 65 |
| | 4 | 22 | 58 | 91 | 24 | 92 | 64 |
| | 5 | 20 | 61 | 89 | 26 | 89 | 59 |
| | 6 | 22 | 68 | 93 | 22 | 93 | 69 |
| | 7 | 24 | 73 | 89 | 25 | 92 | 40 |
| | 12 | 35 | 81 | 96 | 33 | 96 | 72 |
| | 15 | 16 | 47 | 88 | 28 | 96 | 40 |
| | 16 | 19 | 65 | 88 | 25 | 97 | 66 |
| | 17 | 18 | 62 | 82 | 30 | 100 | 49 |
| | 18 | 19 | 61 | 82 | 30 | 100 | 61 |
| | 19 | 20 | 63 | 83 | 28 | 91 | 38 |
| | 20 | 20 | 68 | 93 | 23 | 95 | 70 |
| | 22 | 14 | 55 | 86 | 29 | 100 | 48 |
| | 23 | 18 | 56 | 86 | 30 | 100 | 47 |
| | 24 | 27 | 70 | 92 | 29 | 90 | 60 |
| | 25 | 29 | 69 | 90 | 30 | 91 | 62 |

Test Example 5

Activity on stress ulcers

In each group, 5 Slc: ddy male mice (6 weeks) were employed. Each test compound was kneaded with Tween-80 followed by adding a 0.5 % solution of gum arabic to prepare 30 mg/10 mℓ solution (solution containing 1.5 % Tween-80 and 0.5 % gum arabic). Then, the preparation was administered orally in a rate of 30 mg/kg. After the administration, the mice were restrained and immersed in a water tank of 23 °C for 7 hours. Then, length of the ulcer in the glandular stomach was measured by a stereoscopic microscope (x 10). The results are shown in Table 6.

Table 6

| Compound No. | Inhibition rate (%) |
|---|---|
| 1 | 83.0 |
| 7 | 88.8 |
| 8 | 75.4 |
| 12 | 98.7 |
| 15 | 98.1 |
| 17 | 79.3 |
| 19 | 92.6 |
| 23 | 91.7 |
| 25 | 94.3 |

Test Example 6

Acute toxicity

In each group, 6 Slc: ddy male mice weighing 25 to 29 g (6 weeks) were employed. Each compound

was administered orally thereto and a value of acute toxicity was evaluated with death rate after 7 days.

Table 7

| Compound No. | $LD_{50}$ (mg/kg) |
|---|---|
| 1 | 1000 |
| 6 | 950 |
| 7 | 800 |
| 12 | 1200 |
| 17 | 1000 |
| 24 | 1100 |
| 25 | 1000 |

Test Example 7

Inhibitory activity against kidney disease caused by puromycin aminonucleoside

In each group, 5 Jcl Wistar male mice (7 weeks) were employed. Puromycin aminonucleoside (made by Sigma Chemical Company, U.S.A.) was administered thereto via caudal vein in a dose of 100 mg/kg, and then, a damage was caused on kidney. Each test compound was suspended in a 0.5 % methyl cellose solution to give the concentration such that the dose became 10 mg/kg. The suspension was administered orally thereto 2 times per day for 10 days. Then, content of proteins in the total amount of the urine of 10th day was measured.

The measuring method was the Biuret method by Gornall et al.

Table 8

| Compound No. | Total amount of Urine proteins (mg) |
|---|---|
| 3 | 261.8* ± 38.9 |
| 6 | 235.4* ± 42.5 |
| 7 | 249.3* ± 41.3 |
| 12 | 224.1* ± 31.8 |
| 24 | 251.7* ± 45.8 |
| 25 | 263.4* ± 47.2 |
| 27 | 259.3* ± 50.1 |
| Control (puromycin aminonucleoside) | 394.3 ± 38.9 |

* : significant difference from Control $p< 0.05$

The above Test Examples made clear that the compounds of the present invention were pharmacologically useful. That is, as shown in Test Example 1, the compounds of the present invention showed potent vasodepressor activity and the activity was persistent. As shown in Test Example 2, cerebrocirculation improving activity was evaluated by means of the test of decapitation induced hypoxia in mice as a method for evaluation. As the result of the test, the compounds of the present invention showed remarkable effect. Further, it is known that sympathetic nerves are concerned with relaxation of vascular smooth muscles. As

shown in Test Example 3, the compounds of the present invention showed anti-norepinephrine activity. In circulatory diseases, dynamics of blood is an important factor and particularly, platelet aggregation is greatly concerned with the diseases such as thrombosis and ischemia. For a treatment of these diseases, a platelet aggregation inhibitor is used and as shown in Test Example 4, the compounds of the present invention had potent inhibitory activity against platelet aggregation. Stress ulcers are caused by stress of main desease, and preventing the stress ulcers is significant in clinical medicine. As shown in Test Example 5, the compounds of the present invention showed stress ulcers inhibiting activity. Further, as shown in Test Example 7, the compounds of the present invention had kidney disease inhibiting activity. Furthermore, as is clear from the values of acute toxicity in Test Example 6, the compounds of the present invention were extremely excellent in safety.

Namely, the compounds of the present invention show further potent antihypertensive activity and the effect is persistent. Furthermore, the compounds of the present invention have excellent cerebral blood flow increasing activity, stress ulcers inhibiting activity, coronary blood flow increasing activity, platelet aggregation inhibiting activity, anti-norepinephrine activity, phosphodiesterase (PDE) inhibiting activity and kidney diseases inhibiting activity as pharmacologically useful characteristics.

Therefore, the compounds of the present invention are useful as preventing or therapeutic agents for circulatory diseases, for example, human hypertension, ischemic cardiac diseases such as stenocardia and cardiac infarction, cardiac failure thrombosis and the like, and as preventing or therapeutic agents for kidney diseases, also having stress ulcers inhibiting activity.

As administration forms of the compound having the above-mentioned formula (I), there can be exemplified preparations for oral administration such as tablets, capsules, granules, powders and syrups, preparations for parenteral administration such as subcutaneous injection, intravenous injection, suppositories, cataplasmata and emplastra and the like. These various preparations can be prepared in a usual method by using, besides an effective ingredient, any conventional additives such as excipient, binder, disintegrant, lubricant, corrigent, solubilizer and dispersing agent which can be used in the field of pharmaceutical preparation in accordance with the purpose. The preparation can be prepared by using, for example, gelatin, lactose, sucrose, titanium oxide, starch, crystalline cellulose, hydroxypropylmethylcellulose, carboxymethylcellulose, corn starch, microcrystalline wax, white petrolatum, magnesium alumino meta silicate, anhydrous calcium phosphate, citric acid, trisodium citrate, hydroxypropylcellulose, sorbitol, sorbitan esters of fatty acids, polyvinylpyrrolidone, magnesium stearate, light anhydrous silicic acid, talc, vegetable oil, benzyl alcohol, gum arabic, propylene glycol or polyalkylene glycol.

Although the dosage is different according to sympton, age, body weight and route and times of administration, a usual dosage is about 2 to about 300 mg per day for adults, and can be devided to 1 to several times.

There exists asymmetic carbon in the above-mentioned compound having the formula (I). The present invention includes optical isomers due to asymmetric carbon or a mixture thereof mixed in an optional ratio besides racemic compounds.

Example 1

Preparation of 2,6-dimethyl-4-(3-ethynylphenyl)-3-methoxycarbonyl-5-{4-(6-oxo-1,4,5,6-tetrahydropyridazinon-3-yl)phenyl}aminocarbonyl-1,4-dihydropyridine (compound 1)

In 10 mℓ of isopropanol were dissolved 0.5 g (0.0038 mol) of 3-ethynylbenzaldehyde, 0.46 g (0.004 mol) of methyl 3-aminocrotonate and 1.09 g (0.004 mol) of N-[4-(6-oxo-1,4,5,6-tetrahydropyridazinon-3-yl) phenyl]acetoacetamide with heating and the mixture was refluxed for 3 hours. After standing for cooling, the solvent was distilled away and the obtained residue was subjected to the silica gel column (chloroform : methanol = 93 : 1) to give 1.0 g of the titled compound as pale yellow crystals (yield: 52 %).

m.p.: 129 - 131 °C

MS(m/z) : 480 ($M^+$ - 2)

$^1$H-NMR (δ, ppm) (CDCℓ$_3$):

8.81 (s, 1H), 7.81-7.26 (m, 8H), 6.63 (s, 1H), 6.01 (s, 1H), 4.93 (s, 1H), 3.72 (s, 3H), 3.13 (s, 1H), 2.92 (t, 2H, J = 8Hz), 2.64 (t, 2H, J = 8Hz), 2.33 (s, 3H), 2.30 (s, 3H)

Example 2

Preparation of 2,6-dimethyl-3-methoxycarbonyl-5-{4-(6-oxo-1,4,5,6-tetrahydropyridazinon-3-yl)-phenyl}aminocarbonyl-4-{3-(1-propyn-1-yl)}phenyl-1,4-dihydropyridine (compound 2)

The procedures of reaction, treatment and purification of Example 1 were repeated except for using 0.55 g (0.0038 mol) of {3-(1-propyn-1-yl)}benzaldehyde, 0.46 g (0.004 mol) of methyl 3-aminocrotonate, 1.09 g (0.004 mol) of N-[4-(6-oxo-1,4,5,6-tetrahydropyridazinon-3-yl)phenyl]acetoacetamide and 10 mℓ of isopropanol to give 1.2 g of the titled compound (yield: 58 %).
m.p.: 124 - 126°C
MS(m/z) : 521 ($M^+$)
$^1$H-NMR (δ, ppm) ($CDCℓ_3$):
8.80 (s, 1H), 7.84-7.25 (m, 8H), 6.61 (s, 1H), 6.02 (s, 1H), 4.95 (s, 1H), 3.71 (s, 3H), 2.90 (t, 2H, J=8Hz), 2.64 (t, 2H, J=8Hz), 2.33 (s, 3H), 2.31 (s, 3H), 1.75 (s, 3H)

Example 3

Preparation of 2,6-dimethyl-3-methoxycarbonyl-5-{4-(6-oxo-1,4,5,6-tetrahydropyridazinon-3-yl)-phenyl}aminocarbonyl-4-{3-(1-heptyn-1-yl)}phenyl-1,4-dihydropyridine (compound 3)

The procedures of reaction, treatment and purification of Example 1 were repeated except for using 0.76 g (0.0038 mol) of {3-(1-heptyn-1-yl)}benzaldehyde, 0.46 g (0.004 mol) of methyl 3-aminocrotonate, 1.09 g (0.004 mol) of N-[4-(6-oxo-1,4,5,6-tetrahydropyridazinon-3-yl)phenyl]acetoacetamide and 10 mℓ of isopropanol to give 1.3 g of the titled compound (yield: 56 %).
m.p.: 110 - 112°C
MS(m/z) : 577 ($M^+$)
$^1$H-NMR (δ, ppm) ($CDCℓ_3$):
8.79 (s, 1H), 7.81-7.18 (m, 8H), 6.68 (s, 1H), 6.03 (s, 1H), 5.01 (s, 1H), 3.73 (s, 3H), 2.91 (t, 2H, J=8Hz), 2.66 (t, 2H, J=8Hz), 2.33 (s, 3H), 2.31 (s, 3H), 1.75-1.48 (m, 11H)

Example 4

Preparation of 2,6-dimethyl-3-methoxycarbonyl-5-{4-(6-oxo-1,4,5,6-tetrahydropyridazinon-3-yl)-phenyl}aminocarbonyl-4-[3-{2-(4-nitro)phenyl)ethynyl]phenyl-1,4-dihydropyridine (compound 4)

In 5 mℓ of methylene chloride were suspended 0.433 g (0.001 mol) of 5-carboxy-2,6-dimethyl-3-methoxycarbonyl-4-[3-{2-(4-nitro)phenyl}ethynyl]phenyl-1,4-dihydropyridine under cooling with ice. To this reaction solution was gradually added 0.206 g (0.001 mol) of dicyclohexylcarbodiimide. After stirring under cooling with ice for 30 minutes, to the reaction solution was gradually added 0.189 g (0.001 mol) of 4-(6-oxo-1,4,5,6-tetrahydropyridazinon-3-yl)aniline and the mixture was heated to the room temperature. The precipitated insoluble substance was removed by filtration and 5 mℓ of water was added to the filtrate. Then the layer of methylene chloride was separated. This was washed with 1N HCℓ, saturated aqueous solution of sodium bicarbonate and saturated aqueous solution of sodium chloride, and dried over anhydrous magnesium sulfate. Then the solvent was distilled away and the obtained residue was subjected to silica gel column chromatography (chloroform : methanol = 93 : 1) to give 0.4 g of the titled compound as yellow crystals (yield: 64 %).
m.p.: 105 - 107°C
MS(m/z) : 628 ($M^+$)
$^1$H-NMR (δ, ppm) ($CDCℓ_3$):
8.78 (s, 1H), 7.81-7.19 (m, 12H), 6.68 (s, 1H), 6.01 (s, 1H), 4.93 (s, 1H), 3.69 (s, 3H), 2.91 (t, 2H, J=8Hz), 2.61 (t, 2H, J=8Hz), 2.31 (s, 3H), 2.29 (s, 3H)

Example 5

Preparation of 2,6-dimethyl-3-methoxycarbonyl-5-{4-(6-oxo-1,4,5,6-tetrahydropyridazinon-3-yl)-phenyl}aminocarbonyl-4-{3-(6-morpholino-1-hexyn-1-yl)}phenyl-1,4-dihydropyridine (compound 5)

The procedures of reaction, treatment and purification of Example 1 were repeated except for using 1.03 g (0.0038 mol) of 3-(6-morpholino-1-hexyn-1-yl)benzaldehyde, 0.46 g (0.004 mol) of methyl 3-aminocrotonate, 1.09 g (0.004 mol) of N-[4-(6-oxo-1,4,5,6-tetrahydropyridazinon-3-yl)phenyl]acetoacetamide and 10 mℓ of isopropanol to give 1.4 g of the titled compound (yield: 54 %).
m.p.: 140 - 142°C
MS(m/z) : 648 ($M^+$)

$^1$H-NMR (δ, ppm) (CDCℓ $_3$):
8.79 (s, 1H), 7.81-7.30 (m, 8H), 6.79 (s, 1H), 6.01 (s, 1H), 4.99 (s, 1H), 3.72 (s, 3H), 2.9 (t, 2H, J = 8Hz), 2.61 (t, 2H, J = 8Hz), 2.59-1.54 (m, 16H), 2.34 (s, 3H), 2.31 (s, 3H)

Example 6

Preparation of 2,6-dimethyl-4-{3-(4-hydroxy-1-butyn-1-yl)}phenyl-3-methoxycarbonyl-5-{4-(6-oxo-1,4,5,6-tetrahydropyridazinon-3-yl)phenyl}aminocarbonyl-1,4-dihydropyridine (compound 6)

The procedures of reaction, treatment and purification of Example 1 were repeated except for using 0.66 g (0.0038 mol) of 3-(4-hydroxy-1-butyn-1-yl)benzaldehyde, 0.46 g (0.004 mol) of methyl 3-aminocrotonate, 1.09 g (0.004 mol) of N-[4-(6-oxo-1,4,5,6-tetrahydropyridazinon-3-yl)phenyl]acetoacetamide and 10 mℓ of isopropanol to give 1.1 g of the titled compound (yield: 50 %).
m.p.: 152 - 154 °C
MS(m/z) : 551 ($M^+$)
$^1$H-NMR (δ, ppm) (CDCℓ $_3$):
8.81 (s, 1H), 7.81-7.24 (m, 8H), 6.68 (s, 1H), 6.63 (s, 1H), 6.03 (s, 1H), 5.01 (s, 1H), 3.73 (s, 3H), 2.89 (t, 2H, J = 8Hz), 2.64 (t, 2H, J = 8Hz), 2.34 (s, 3H), 2.31 (s, 3H), 1.81-1.85 (m, 4H)

Example 7

Preparation of 2,6-dimethyl-4-{3-(3-hydroxy-3-methyl)-1-butyn-1-yl)}phenyl-3-methoxycarbonyl-5-{4-(6-oxo-1,4,5,6-tetrahydropyridazinon-3-yl)phenyl}aminocarbonyl-1,4-dihydropyridine (compound 7)

The procedures of reaction, treatment and purification of Example 1 were repeated except for using 0.71 g (0.0038 mol) of 3-{(3-hydroxy-3-methyl)-1-butyn-1-yl}benzaldehyde, 0.46 g (0.004 mol) of methyl 3-aminocrotonate, 1.09 g (0.004 mol) of N-[4-(6-oxo-1,4,5,6-tetrahydropyridazinon-3-yl)phenyl]acetoacetamide and 10 mℓ of isopropanol to give 1.3 g of the titled compound (yield: 58 %).
m.p.: 144 - 146 °C
MS(m/z) : 565 ($M^+$)
$^1$H-NMR (δ, ppm) ($CDCl_3$):
8.55 (s, 1H), 7.71-7.21 (m, 8H), 6.89 (s, 1H), 6.54 (s, 1H), 5.82 (s, 1H), 4.85 (s, 1H), 3.72 (s, 3H), 2.92 (t, 2H, J = 8Hz), 2.55 (t, 2H, J = 8Hz), 2.33 (s, 3H), 2.31 (s, 3H), 1.63 (s, 3H), 1.62 (s, 3H)

Example 8

Preparation of 4-{3-(2-tert-butoxycarbonyl)ethynyl}phenyl-2,6-dimethyl-5-{4-(6-oxo-1,4,5,6-tetrahydropyridazinon-3-yl)phenyl}aminocarbonyl-3-methoxycarbonyl-1,4-dihydropyridine (compound 8)

The procedures of reaction, treatment and purification of Example 1 were repeated except for using 0.874 g (0.0038 mol) of 3-(2-tert-butoxycarbonyl)ethynyl benzaldehyde, 0.46 g (0.004 mol) of methyl 3-aminocrotonate, 1.09 g (0.004 mol) of N-[4-(6-oxo-1,4,5,6-tetrahydropyridazinon-3-yl)phenyl]acetoacetamide and 10 mℓ of isopropanol to give 1.2 g of the titled compound (yield: 49 %).
m.p.: 102 - 104 °C
MS(m/z) : 607 ($M^+$)
$^1$H-NMR (δ, ppm) (CDCℓ $_3$):
8.83 (s, 1H), 7.81-7.21 (m, 8H), 6.88 (s, 1H), 6.0 (s, 1H), 5.01 (s, 1H), 3.69 (s, 3H), 2.90 (t, 2H, J = 8Hz), 2.65 (t, 2H, J = 8Hz), 2.32 (s, 3H), 2.31 (s, 3H), 1.51 (s, 9H)

Example 9

Preparation of 4-(3-chloro-2-ethynyl)phenyl-2,6-dimethyl-3-methoxycarbonyl-5-{4-(6-oxo-1,4,5,6-tetrahydropyridazinon-3-yl)phenyl}aminocarbonyl-1,4-dihydropyridine (compound 9)

In 20 mℓ of isopropanol were dissolved 1.64 g (0.01mol) of 3-chloro-2-ethynylbenzaldehyde, 1.16 g (0.01 mol) of methyl acetoacetate and 2.72 g (0.01 mol) of N-[4-(6-oxo-1,4,5,6-tetrahydropyridazinon-3-yl)-phenyl] aminocrotonamide on heating and the mixture was refluxed for 6 hours. After standing for cooling, the solvent was distilled away and the obtained residue was subjected to the silica gel column chromatography (chlorform : methanol = 93: 1) to give 2.6 g of the titled compound as yellow crystals (yield: 50 %).
m.p.: 118 - 120° C
MS(m/z) : 516 ($M^+$)
$^1$H-NMR (δ, ppm) (CDCℓ 3):
8.79 (s, 1H), 7.80-7.25 (m, 7H), 6.87 (s, 1H), 5.99 (s, 1H), 4.92 (s, 1H), 3.73 (s, 3H), 3.11 (s, 1H), 2.91 (t, 2H, J=8Hz), 2.63 (t, 2H, J=8Hz), 2.30 (s, 3H), 2.31 (s, 3H)

Example 10

Preparation of 3-cyclopropylaminocarbonyl-2,6-dimethyl-4-(3-ethynylphenyl)-5-{4-(6-oxo-1,4,5,6-tetrahydropyridazinon-3-yl)phenyl}aminocarbonyl-1,4-dihydropyridine (compound 10)

The procedures of reaction, treatment and purification of Example 1 were repeated except for using 0.49 g (0.0038 mol) of 3-ethynylbenzaldehyde, 0.568 g (0.004 mol) of (2-amino-4-cyclopropylamino)-2-buten-4-one, 1.09 g (0.004 mol) of N-[4-(6-oxo-1,4,5,6-tetrahydropyridazinon-3-yl)phenyl]acetoacetamide and 10 mℓ of isopropanol to give 1.5 g of the titled compound (yield: 74 %).
m.p.: 192 - 194° C
MS(m/z) : 507 ($M^+$)
$^1$H-NMR (δ, ppm) (CDCℓ 3):
10.8 (s, 1H), 9.42 (s, 1H), 8.30 (s, 1H), 7.65-7.18 (m, 8H), 6.69 (s, 1H), 4.95 (s, 1H), 3.20 (s, 1H), 2.95 (t, 2H, J=8Hz), 2.43 (t, 2H, J=8Hz), 2.12 (s, 3H), 2.02 (s, 3H), 0.51-0.21 (m, 5H)

Example 11

Preparation of 2,6-dimethyl-4-(3-ethynylphenyl)-3-(N-methyl-N-propyl)aminocarbonyl-5-{4-(6-oxo-1,4,5,6-tetrahydropyridazinon-3-yl)phenyl}aminocarbonyl-1,4-dihydropyridine (compound 11)

The procedures of reaction, treatment and purification of Example 1 were repeated except for using 0.49 g (0.0038 mol) of 3-ethynylbenzaldehyde, 0.62 g (0.004 mol) of N-methyl-N-propyl-3-aminocrotonamide, 1.09 g (0.004 mol) of N-[4-(6-oxo-1,4,5,6-tetrahydropyridazinon-3-yl)phenyl]-acetoacetamide and 10 mℓ of isopropanol to give 1.4 g of the desired compound (yield: 67 %).
m.p.: 185 - 187° C
MS(m/z) : 521 ($M^+$)
$^1$H-NMR (δ, ppm) (CDCℓ 3):
9.32 (s, 1H), 8.52 (s, 1H), 7.66-7.20 (m, 8H), 6.83 (s, 1H), 4.91 (s, 1H), 3.22 (s, 1H), 2.93 (t, 2H, J=8Hz), 2.8 (s, 3H), 2.72-2.34 (m, 7H), 2.43 (t, 2H, J=8Hz), 2.13 (s, 3H), 2.0 (s, 3H)

Example 12

Preparation of 2-amino-3-ethoxycarbonyl-4-(3-ethynylphenyl)-6-methyl-5-{4-(6-oxo-1,4,5,6-tetrahydropyridazinon-3-yl)phenyl)aminocarbonyl-1,4-dihydropyridine (compound 12)

In 200 mℓ of ethanol was dissolved 9.3 g (0.024 mol) of N-[4-(6-oxo-1,4,5,6-tetrahydropyridazinon-3-yl) phenyl]3-ethynylbenzylideneacetoacetamide. Thereto were added 4.0 g (0.024 mol) of ethyl 3,3-diamino-2-propenoate hydrochloride and 1.63 g (0.024 mol) of sodium ethoxide. The mixture was heated under reflux for 1 hour. After standing for cooling, the solvent was distilled away and the obtained residue was subjected to silica gel column chromatography (chloroform : methanol = 10 : 0.4) to give 10.1 g of the titled compound as orange crystals (yield: 85 %).
m.p.: 170 - 173° C
MS(m/z) : 497 ($M^+$)
$^1$H-NMR (δ, ppm) (CDCℓ 3):
10.83 (s, 1H), 9.68 (s, 1H), 8.3 (brs, 2H), 7.67-7.19 (m, 8H), 6.73 (brs, 1H), 4.82 (s, 1H), 3.97-3.89 (q, 2H, J=7Hz), 3.36 (s, 1H), 2.90 (t, 2H, J=8Hz), 2.42 (t, 2H, J=8Hz), 2.05 (s, 3H), 1.05 (t, 3H, J=7Hz)

Example 13

Preparation of 2,6-dimethyl-4-(3-ethynylphenyl)-3-methoxycarbonyl-5-{4-(1-methyl-6-oxo-1,4,5,6-tetrahydropyridazinon-3-yl)phenyl}aminocarbonyl-1,4-dihydropyridine (compound 13)

The procedures of reaction, treatment and purification of Example 1 were repeated except for using 0.67 g (0.0052 mol) of 3-ethynylbenzaldehyde, 0.79 g (0.0052 mol) of methyl 3-aminocrotonate, 1.50 g (0.0052 mol) of N-[4-(1-methyl-6-oxo-1,4,5,6-tetrahydropyridazinon-3-yl)phenyl]acetoacetamide and 30 mℓ of isopropanol to give 1.02 g of the titled compound (yield: 37 %).
m.p.: 191 - 193 ° C
MS(m/z) : 532 ($M^+$)
$^1$H-NMR (δ, ppm) (CDCℓ $_3$):
9.37 (s, 1H), 7.73-7.27 (m, 8H), 6.80 (s, 1H), 6.02 (s, 1H), 4.85 (s, 1H), 3.61 (s, 3H), 3.45 (s, 3H), 2.93 (t, 2H, J = 8Hz), 2.57 (t, 2H, J = 8Hz), 2.34 (s, 3H), 2.31 (s, 3H)

Example 14

Preparation of 2,6-dimethyl-4-(3-ethynylphenyl)-3-methoxycarbonyl-5-{4-(1-ethyl-6-oxo-1,4,5,6-tetrahydropyridazinon-3-yl)phenyl}aminocarbonyl-1,4-dihydropyridine (compound 14)

The procedures of reaction, treatment and purification of Example 1 were repeated except for using 0.49 g (0.0038 mol) of 3-ethynylbenzaldehyde, 0.46 g (0.004 mol) of methyl 3-aminocrotonate, 1.20 g (0.004 mol) of N-[4-(1-ethyl-6-oxo-1,4,5,6-tetrahydropyridazinon-3-yl)phenyl]acetoacetamide and 10 mℓ of isopropanol to give 1.1 g of the titled compound (yield: 50 %).
m.p.: 123 - 125 ° C
MS(m/z) : 546 ($M^+$)
$^1$H-NMR (δ, ppm) (CDCℓ $_3$):
9.31 (s, 1H), 7.70-7.27 (m, 8H), 6.82 (s, 1H), 6.02 (s, 1H), 4.95 (s, 1H), 3.69 (s, 3H), 2.91 (t, 2H, J = 8Hz), 2.78 (q, 2H, J = 7Hz), 2.55 (t, 2H, J = 8Hz), 2.33 (s, 3H), 2.31 (s, 3H), 1.55 (t, 3H, J = 7Hz)

Example 15

Preparation of 2,6-dimethyl-4-(3-ethynylphenyl)-3-methoxycarbonyl-5-{4-(5-methyl-6-oxo-1,4,5,6-tetrahydropyridazinon-3-yl)phenyl}aminocarbonyl-1,4-dihydropyridine (compound 15)

The procedures of reaction, treatment and purification of Example 1 were repeated except for using 0.49 g (0.0038 mol) of 3-ethynylbenzaldehyde, 0.46 g (0.004 mol) of methyl 3-aminocrotonate, 1.15 g (0.004 mol) of N-[4-(5-methyl-6-oxo-1,4,5,6-tetrahydropyridazinon-3-yl)phenyl]acetoacetamide and 10 mℓ of isopropanol to give 1.33 g of the titled compound (yield: 65 %).
m.p.: 125 - 127 ° C
MS(m/z) : 511 ($M^+$)
$^1$H-NMR (δ, ppm) (CDCℓ $_3$):
8.82 (s, 1H), 7.79-7.27 (m, 8H), 6.67 (s, 1H), 6.02 (s, 1H), 4.95 (s, 1H), 3.73 (s, 3H), 3.11 (s, 1H), 2.89, 2.86 (dd, 2H, J = 6.5Hz), 2.61 (m, 1H), 2.32 (s, 3H), 2.31 (s, 3H), 1.12 (d, 3H, J = 6.5Hz)

Example 16

Preparation of 2,6-dimethyl-4-(3-ethynylphenyl)-3-methoxycarbonyl-5-{4-(4-methyl-6-oxo-1,4,5,6-tetrahydropyridazinon-3-yl)phenyl}aminocarbonyl-1,4-dihydropyridine (compound 16)

The procedures of reaction, treatment and purification of Example 1 were repeated except for using 0.49 g (0.0038 mol) of 3-ethynylbenzaldehyde, 0.46 g (0.004 mol) of methyl 3-aminocrotonate, 1.15 g (0.004 mol) of N-[4-(4-methyl-6-oxo-1,4,5,6-tetrahydropyridazinon-3-yl)phenyl]acetoacetamide and 10 mℓ of isopropanol to give 1.5 g of the titled compound (yield: 73 %).
m.p.: 126 - 127 ° C
MS(m/z) : 511 ($M^+$)
$^1$H-NMR (δ, ppm) (CDCℓ $_3$):
8.81 (s, 1H), 7.78-7.26 (m, 8H), 6.80 (s, 1H), 6.02 (s, 1H), 4.96 (s, 1H), 3.72 (s, 3H), 3.10 (s, 1H), 2.88, 2.86 (dd, 2H, J = 6.5Hz), 2.62 (m, 1H), 2.32, (s, 3H), 2.31 (s, 3H), 1.07 (d, 3H, J = 6.5Hz)

Example 17

Preparation of 2,6-dimethyl-4-{3-(3-hydroxy-3-methyl)-1-butyn-1-yl-6-methoxy}phenyl-3-ethoxycarbonyl-5-{4-(6-oxo- 1,4,5,6-tetrahydropyridazinon-3-yl)phenyl}aminocarbonyl-1,4-dihydropyridine (compound 17)

The procedures of reaction, treatment and purification of Example 1 were repeated except for using 0.83 g (0.0038 mol) of {3-(3-hydroxy-3-methyl)-1-butyn-1-yl}-6-methoxybenzaldehyde, 0.52 g (0.004 mol) of ethyl 3-aminocrotonate, 1.09 g (0.004 mol) of N-[4-(6-oxo-1,4,5,6-tetrahydropyridazinon-3-yl)phenyl]-acetoacetamide and 10 mℓ of isopropanol to give 1.1 g of the titled compound (yield: 50 %).
m.p.: 151 - 153 ° C
MS(m/z) : 584 ($M^+$)
$^1$H-NMR (δ, ppm) ($CDCℓ_3$):
9.03 (s, 1H), 7.71-7.22 (m, 7H), 6.61 (s, 1H), 6.34 (s, 1H), 5.02 (s, 1H), 4.05 (s, 3H), 4.79 (s, 1H), 4.07 (q, 2H, J = 7Hz), 2.90 (t, 2H, J = 8Hz), 2.56 (t, 2H, J = 8Hz), 2.34 (s, 3H), 2.31 (s, 3H) 1.63 (s, 3H), 1.62 (s, 3H), 1.23 (t, 3H, J = 7Hz)

Example 18

Preparation of 2,6-dimethyl-4-(3-ethynyl-6-methoxyphenyl)-3-ethoxycarbonyl-5-{4-(6-oxo-1,4,5,6-tetrahydropyridazinon-3-yl)phenyl}aminocarbonyl-1,4-dihydropyridine (compound 18)

The procedures of reaction, treatment and purification of Example 1 were repeated except for using 0.78 g (0.0038 mol) of 3-ethynyl-6-methoxybenzaldehyde, 0.52 g (0.004 mol) of ethyl 3-aminocrotonate, 1.09 g (0.004 mol) of N-[4-(6-oxo-1,4,5,6-tetrahydropyridazinon-3-yl)phenyl]acetoacetamide and 10 mℓ of isopropanol to give 1.2 g of the titled compound (yield: 58 %).
m.p.: 138 - 140 ° C
MS(m/z) : 526 ($M^+$)
$^1$H-NMR (δ, ppm) ($CDCℓ_3$):
9.01 (s, 1H), 7.70-7.24 (m, 7H), 6.61 (s, 1H), 6.34 (s, 1H), 4.05 (s, 3H), 4.79 (s, 1H), 4.07 (q, 2H, J = 7Hz), 3.07 (s, 1H), 2.90 (t, 2H, J = 8Hz), 2.56 (t, 2H, J = 8Hz), 2.33 (s, 3H), 2.31 (s, 3H), 1.23 (t, 3H, J = 7Hz)

Example 19

Preparation of 2,6-dimethyl-4-(3-ethynylphenyl)-5-{4-(4-ethyl-6-oxo-1,4,5,6-tetrahydropyridazinon-3-yl)-phenyl} aminocarbonyl-3-methoxycarbonyl-1,4-dihydropyridine (compound 19)

The procedure of reaction, treatment and purification of Example 1 were repeated except for using 0.25 g (0.0019 mol) of 3-ethynylbenzaldehyde, 0.22 g (0.0019 mol) of methyl 3-aminocrotonate, 0.56 g (0.0019 mol) of N-[4-(4-ethyl-6-oxo-1,4,5,6-tetrahydropyridazinon-3-yl)phenyl]acetoacetamide and 20 mℓ of isopropanol to give 0.6 g of the titled compound (yield: 62 %).
m.p.: 116 - 118 ° C
MS(m/z) : 510 ($M^+$)
$^1$H-NMR (δ, ppm) ($CDCℓ_3$):
8.42 (s, 1H), 7.75-7.19 (m, 9H), 5.67 (s, 1H), 4.85 (s, 1H), 3.69 (s, 3H), 3.14-3.02 (m, 2H), 2.67-2.61 (m, 3H), 2.36 (s, 3H), 2.32 (s, 3H), 0.97 (t, 3H, J = 7Hz)

Example 20

Preparation of 2,6-dimethyl-4-(3-ethynylphenyl)-3-ethoxycarbonyl-5-{4-(6-oxo-1,4,5,6-tetrahydropyridazinon-3-yl)phenyl}aminocarbonyl-1,4-dihydropyridine (compound 20)

The procedures of reaction, treatment and purification of Example 1 were repeated except for using 0.5 g (0.0038 mol) of 3-ethynylbenzaldehyde, 0.5 g (0.0038 mol) of ethyl 3-aminocrotonate, 1.0 g (0.0037 mol) of N-[4-(6-oxo-1,4,5,6-tetrahydropyridazinon-3-yl)phenyl] acetoacetamide and 30 mℓ of isopropanol to give 1.0 g of the titled compound (yield: 55 %).
m.p.: 127 - 128 ° C
MS(m/z) : 497 ($M^+$)
$^1$H-NMR (δ, ppm) ($CDCℓ_3$):
8.49 (s, 1H), 7.72-7.20 (m, 9H), 5.68 (s, 1H), 4.84 (s, 1H), 4.15 (q, 2H, J = 7Hz), 3.09 (s, 1H), 2.94 (t, 2H, J = 8Hz), 2.61 (t, 2H J = 8Hz), 2.35 (s, 3H), 2.33 (s, 3H), 1.27 (t, 3H, J = 7Hz)

Example 21

Preparation of 2,6-dimethyl-4-(3-ethynylphenyl)-3-methoxycarbonyl-5-{4-(6-oxo-4-propyl-1,4,5,6-tetrahydropyridazinon-3-yl)phenyl}aminocarbonyl-1,4-dihydropyridine (compound 21)

The procedures of reaction, treatment and purification of Example 1 were repeated except for using 2 g (0.015 mol) of 3-ethynylbenzaldehyde, 1.8 g (0.015 mol) of methyl 3-aminocrotonate, 4.5 g (0.014 mol) of N-[4-(6-oxo-4-propyl-1,4,5,6-tetrahydropyridazinon-3-yl)phenyl]acetoacetamide and 50 mℓ of isopropanol to give 3.5 g of the titled compound (yield: 48 %).
m.p.: 112 - 114 ° C
MS(m/z) : 525 ($M^+$)
$^1$H-NMR (δ, ppm) (CDCℓ $_3$):
8.60 (s, 1H), 7.71-7.23 (m, 9H), 5.85 (s, 1H), 4.86 (s, 1H), 3.68 (s, 3H), 3.21-3.19 (m, 1H), 3.10 (s, 1H), 2.68-2.50 (m, 2H), 2.36 (s, 3H), 2.32 (s, 3H), 1.61-1.10 (m, 4H), 0.88 (t, 3H, J = 7Hz)

Example 22

Preparation of 2,6-dimethyl-4-(3-ethynylphenyl)-3-methoxyethoxycarbonyl-5-{4-(6-oxo-1,4,5,6-tetrahydropyridazinon-3-yl)phenyl}aminocarbonyl-1,4-dihydropyridine (compound 22)

The procedures of reaction, treatment and purification of Example 1 were repeated except for using 0.37 g (0.0028 mol) of 3-ethynylbenzaldehyde, 0.42 g (0.0026 mol) of methoxyethyl 3-aminocrotonate, 0.7 g (0.0026 mol) of N-[4-(6-oxo-1,4,5,6-tetrahydropyridazinon-3-yl)phenyl]acetoacetamide and 20 mℓ of isopropanol to give 0.85 g of the titled compound (yield: 62 %).
m.p.: 100 - 102 ° C
MS(m/z) : 527 ($M^+$)
$^1$H-NMR (δ, ppm) (CDCℓ $_3$):
8.46 (s, 1H), 7.72-7.20 (m, 9H), 5.66 (s, 1H), 4.86 (s, 1H), 4.31-4.13 (m, 2H), 3.62-3.57 (m, 2H), 3.38 (s, 3H), 3.10 (s, 1H), 2.9 (t, 2H, J = 7Hz), 2.63 (t, 2H, J = 7Hz), 2.36 (s, 3H), 2.30 (s, 3H)

Example 23

Preparation of 2-amino-4-(3-ethynylphenyl)-3-ethoxycarbonyl-6-methyl-5-{4-(6-oxo-4-methyl-1,4,5,6-tetrahydropyridazinon-3-yl)phenyl}aminocarbonyl-1,4-dihydropyridine (compound 23)

The procedures of reaction, treatment and purification of Example 12 were repeated except for using 0.9 g (0.0055 mol) of ethyl 3,3-diamino-2-propenoate hydrochloride, 0.4 g (0.0055 mol) of sodium ethoxide, 2.2 g (0.0055 mol) of N-[4-(6-oxo-4-methyl-1,4,5,6-tetrahydropyridazinon-3-yl)phenyl]-3-ethynylbenzylideneacetoacetamide and 50 mℓ of ethanol to give 0.8 g of the titled compound (yield: 28.6 %).
m.p.: 165 - 167 ° C
MS(m/z) : 511 ($M^+$)
$^1$H-NMR (δ, ppm) (CDCℓ $_3$):
10.71 (s, 1H), 9.5 (s, 1H), 8.1 (brs, 2H), 7.66-7.1 (m, 8H), 6.73 (brs, 1H), 4.79 (s, 1H), 3.9-3.81(q, 2H), 3.3 (s, 1H), 2.73, 2.66 (dd, 2H, J = 6.5Hz), 2.6 (m, 1H), 2.34 (s, 3H), 1.08 (d, 3H, J = 6.5Hz), 1.05 (t, 3H)

Example 24

Preparation of 2-amino-4-{3-(3-hydroxy-3-methyl)-1-butyn-1-yl}phenyl-3-ethoxycarbonyl-6-methyl-5-{4-(6-oxo-1,4,5,6-tetrahydropyridazinon-3-yl)phenyl}aminocarbonyl-1,4-dihydropyridine (compound 24)

The procedures of reaction, treatment and purification of Example 12 were repeated except for using 0.9 g (0.0055 mol) of ethyl 3,3-diamino-2-propenoate hydrochloride, 0.4 g (0.0055 mol) of sodium ethoxide, 2.42 g (0.0055 mol) of N-[4-(6-oxo-1,4,5,6-tetrahydropyridazinon-3-yl)phenyl]-3-{(3-hydroxy-3-methyl)-1-butyn-1-yl}benzylideneacetoacetamide and 50 mℓ of ethanol were used to give 1.1 g of the titled compound (yield: 36 %).
m.p.: 160 - 162 ° C
MS(m/z) : 555 ($M^+$)
$^1$H-NMR (δ, ppm) (CDCℓ $_3$):

10.5 (s, 1H), 9.3 (s, 1H), 8.1 (brs, 2H), 7.7-7.1 (m, 8H), 6.72 (brs, 1H), 6.7 (s, 1H), 4.9 (s, 1H), 3.7-3.6 (q, 2H), 2.9 (t, 2H, J = 8Hz), 2.6 (t, 2H, J = 8Hz), 2.32 (s, 3H), 1.62 (s, 3H), 1.61 (s, 3H), 1.2 (t, 3H)

Example 25

Preparation of 2-amino-4-(3-ethynylphenyl)-3-methoxyethoxycarbonyl-6-methyl-5-{4-(4-ethyl-6-oxo-1,4,5,6-tetrahydropyridazinon-3-yl)phenyl}aminocarbonyl-1,4-dihydropyridine (compound 25)

The procedures of reaction, treatment and purification of Example 12 were repeated except that 9.98 g (0.024 mol) of N-[4-(4-ethyl-6-oxo-1,4,5,6-tetrahydropyridazinon-3-yl)phenyl]-3-ethynylbenzylideneacetoacetamide was dissolved in 200 mℓ of ethanol and 4.7 g (0.024 mol) of Methoxyethyl 3,3-diamino-2-propenoate hydrochloride and 1.63g (0.024 mol) of sodium ethoxide were used, to give 12 g of the titled compound (yield: 91 %).
m.p.: 140 - 143 ° C
MS(m/z) : 555 ($M^+$)
$^1$H-NMR (δ, ppm) ($CDCℓ_3$):
10.7 (s, 1H), 9.2 (s, 1H), 8.3 (brs, 2H), 7.8-7.2 (m, 8H), 6.6 (brs, 1H), 5.0 (s, 1H), 4.3-4.2 (m, 2H), 3.81 (s, 3H), 3.62-3.57 (m, 2H), 3.14-3.02 (m, 2H), 2.67-2.61 (m, 3H), 2.4 (s, 3H), 0.96 (t, 3H, J = 7Hz)

Example 26

Preparation of 2-amino-3-cyclopropylaminocarbonyl-4-(3-ethynylphenyl)-6-methyl-5-{4-(5-methyl-6-oxo-1,4,5,6- tetrahydropyridazinon-3-yl)phenyl}aminocarbonyl-1,4-dihydropyridine (compound 26)

The procedures of reaction, treatment and purification of Example 12 were repeated except for using 4.2 g (0.024 mol) of N-cyclopropyl-3,3-diamino-2-propenamide hydrochloride, 1.63 g (0.024 mol) of sodium ethoxide, 9.62 g (0.024 mol) of N-[4-(5-methyl-6-oxo-1,4,5,6-tetrahydropyridazinon-3-yl)phenyl]-3-ethynylbenzylideneacetoacetamide and 200 mℓ of ethanol to give 10.4 g of the titled compound (yield: 83 %).
m.p.: 170 - 172 ° C
MS(m/z) : 520 ($M^+$)
$^1$H-NMR (δ, ppm) ($CDCℓ_3$):
10.7 (s, 1H), 9.4 (s, 1H), 8.29 (s, 1H), 8.2 (brs, 2H), 5.7 (s, 1H), 4.8 (s, 1H), 3.1 (s, 1H), 2.88-2.86 (m, 2H), 2.61 (m, 1H), 2.3 (s, 3H), 0.9 (d, 3H), 0.51-0.2 (m, 5H)

Formulation Examples of the compound of the present invention are shown as follows.

Formulation Example 1

In a centrifugal fluidizing type coating apparatus (DF-360, made by Freund Industrial Co., Ltd.) was introduced 1620 g of lactose (100 mesh, from DMV De Melkindustrie Veghel Co., Ltd., Holland). Thereon was sprayed for coating 1000 mℓ of ethanol/methylene chloride (1:1 v/v) solution in which 30 g of the compound No. 1 and 310 g of hydroxypropylmethylcellulose 2910 (HPMC 2910, from The Shin-etsu Chemical Co., Ltd.) were completely dissolved, to obtain granules according to a conventional process. After drying them at 40 ° C for 4 hours, they were granulated to give a granule according to a conventional process.

Formulation Example 2

In a centrifugal fluidizing type coating apparatus (DF-360, made by Freund Industrial Co., Ltd.) was introduced 1650 g of lactose (100 mesh, from DMV De Melkindustrie Veghel Co., Ltd. Holland). Thereon was sprayed for coating 5000 mℓ of ethanol/methylene chloride (1:1 v/v) solution in which 30 g of the compound No. 1 and 310 g of hydroxypropylmethylcellulose 2910 (HPMC 2910, from The Shin-etsu Chemical Co., Ltd.) were completely dissolved, to obtain granules according to a conventional process. After drying them at 40 ° C for 4 hours, they were granulated according to a conventional process. After 3 g of magnesium stearate was added thereto followed by mixing, they were filled into capsules to give 1000 capsules which contain 30 mg of the compound No. 1 per capsule.

Formulation Example 3

In 200 mℓ of ethanol were dissolved 3 g of the compound No. 1 and 33 g of polyvinylpyrrolidone, and then ethanol was distilled away with drying under reduced pressure. The residue was pulverized to powder. Thereto were added 22 g of lactose, 21 g of calcium carboxymethylcellulose and 1 g of magnesium stearate. According to a conventional process, the mixture was tabletted to give 1000 tablets which contain 3 mg of the compound No. 1 per tablet.

Formulation Example 4

The powder obtained in Example 3, 52 g of corn starch, 62 g of lactose and starch paste were mixed to give granules. Thereto was added 2 g of magnesium stearate, and the mixture was tabletted according to a conventional process to give sublingual tablets containing 3 mg of the compound No. 1 per tablet.

Formulation Example 5

To the mixture solution of 52 g of microcrystalline wax and 104 g of paraffin which were fused with heating was added 41 g of white soft paraffine, and the mixture was kneaded and transferred into a chaser mill. Separately, isopropyl myristate solution containing 3 g of the compound No. 1 was prepared. The prepared solution was gradually added to the mixture with stirring. The mixture was kneaded to give an ointment.

Formulation Example 6

In 150 mℓ of 90 % ethanol was dissolved 0.36 g of the compound No. 1. Then the solution was added to the distilled water for injection containing 150 mℓ of propyleneglycol, 2 g of sodium citrate and 0.3 g of citric acid to give total amount of 600 mℓ. The solution was filtrated with sterilized filter and filled into sterilized vials followed by plugging with sterilized gum stoppers. Each vial contains 5 mℓ of injection solution whose concentration was 0.6 mg/mℓ.

Formulation Example 7

Various components consisting of 3 g of the compound No. 1, 25 g of polyvinylpyrrolidone, 5 g of polyethyleneglycol 400, 25 g of magnesium alumino meta silicate, 138 g of a mixture of starch and anhydrous calcium phosphate (8:2) and 1 g of magnesium stearate were mixed in such proportion and compressed with shaping to give tablets containing 3 mg of the compound per tablet according to a conventional process.

**Claims**

**1.** An ethynylphenyl derivative substituted by pyridazinone having the formula (I):

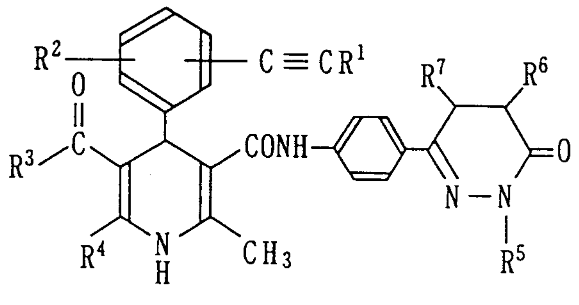

(I)

wherein $R^1$ is hydrogen atom, a $C_{1-6}$ alkyl group, a $C_{1-4}$ alkoxycarbonyl group, a substituted amino $C_{1-6}$ alkyl group, a hydroxy $C_{1-4}$ alkyl group or a phenyl group substituted by an alkyl group, an alkoxyl group, nitro group or a halogen atom, $R^2$ is hydrogen atom, a $C_{1-4}$ alkoxyl group, or a halogen atom, $R^3$ is a $C_{1-4}$ alkoxyl group, a $C_{1-6}$ alkylamino group or a $C_{1-4}$ alkoxy $C_{1-4}$ alkoxyl group, $R^4$ is a $C_{1-4}$ alkyl group or amino group, $R^5$ is hydrogen atom or a $C_{1-4}$ alkyl group, $R^6$ is hydrogen atom or a $C_{1-4}$ alkyl group, and $R^7$ is hydrogen atom or a $C_{1-4}$ alkyl group, which may be a racemate or an optical isomer, or a pharmacologically acceptable salt thereof.

2. An ethynylphenyl derivative substituted by pyridazinone or a pharmacologically acceptable salt thereof of claim 1, wherein $R^1$, $R^2$, $R^5$, $R^6$ and $R^7$ are hydrogen atom, $R^3$ is a $C_{1-4}$ alkoxyl group and $R^4$ is a $C_{1-4}$ alkyl group or amino group in the formula (I).

3. An ethynylphenyl derivative substituted by pyridazinone or a pharmacologically acceptable salt thereof of claim 1, wherein the compound having the formula (I) is 2,6-dimethyl-4-(3-ethynylphenyl)-3-methoxycarbonyl-5-{4-(6-oxo-1,4,5,6-tetrahydropyridazinon-3-yl)phenyl}aminocarbonyl-1,4-dihydropyridine.

4. An ethynylphenyl derivative substituted by pyridazinone or a pharmacologically acceptable salt thereof of claim 1, wherein the compound having the formula (I) is 2-amino-3-ethoxycarbonyl-4-(3-ethynylphenyl)-6-methyl-5-{4-(6-oxo-1,4,5,6-tetrahydropyridazinon-3-yl)phenyl}aminocarbonyl-1,4-dihydropyridine.

5. A medicament for a circulatory disease comprising an ethynylphenyl derivative substituted by pyridazinone or a pharmacologically acceptable salt thereof of claim 1 as an effective ingredient.

6. A medicament for thrombosis comprising an ethynylphenyl derivative substituted by pyridazinone or a pharmacologically acceptable salt thereof of claim 1 as an effective ingredient.

7. A medicament for a kidney disease comprising an ethynylphenyl derivative substituted by pyridazinone or a pharmacologically acceptable salt thereof of claim 1 as an effective ingredient.

8. A medicament for a circulatory disease comprising an ethynylphenyl derivative substituted by pyridazinone or a pharmacologically acceptable salt thereof of claim 2 as an effective ingredient.

9. A medicament for thrombosis comprising an ethynylphenyl derivative substituted by pyridazinone or a pharmacologically acceptable salt thereof of claim 2 as an effective ingredient.

10. A medicament for a kidney disease comprising an ethynylphenyl derivative substituted by pyridazinone or a pharmacologically acceptable salt thereof of claim 2 as an effective ingredient.

# INTERNATIONAL SEARCH REPORT

International Application No PCT/JP91/01316

**I. CLASSIFICATION OF SUBJECT MATTER** (if several classification symbols apply, indicate all) [6]

According to International Patent Classification (IPC) or to both National Classification and IPC

Int. $Cl^5$ C07D401/12, A61K31/50

**II. FIELDS SEARCHED**

Minimum Documentation Searched [7]

| Classification System | Classification Symbols |
|---|---|
| IPC | C07D401/12, A61K31/50 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 61-145180 (Bayer AG), July 2, 1986 (02. 07. 86) & EP, A2, 185964 & US, A, 4707479 | 1-10 |
| A | JP, A, 58-69868 (Mitsui Toatsu Chemicals, Inc.), April 26, 1983 (26. 04. 83) & EP, A1, 107735 & US, A, 4639451 | 1-10 |
| A | JP, A, 59-227860 (Bayer AG), December 21, 1984 (21. 12. 84) & EP, A2, 127826 & US, A, 4540684 | 1-10 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| November 22, 1991 (22. 11. 91) | December 9, 1991 (09. 12. 91) |
| **International Searching Authority** | **Signature of Authorized Officer** |
| Japanese Patent Office | |